# EUROPEAN PATENT APPLICATION

(11) **EP 1 148 124 A1**
(43) Date of publication of application: **24.10.2001**
(21) Application number: 00900334.4
(22) Date of filing: 05.01.2000
(51) Int. Cl.: C12N 15/12, C12N 5/10, G01N 33/50, G01N 33/15

(54) **CELLS FOR MEASURING ABILITY TO PROMOTE TRANSCRIPTION**

(30) Priority: 08.01.1999 JP 280799
(71) Applicant: SUMITOMO CHEMICAL COMPANY LIMITED, Osaka-shi, Osaka 541-0041 (JP)
(72) Inventor: MATSUNAGA, Haruyuki, Osaka-shi, Osaka 552-0004 (JP); OOE, Norihisa, Osaka-shi, Osaka 530-0041 (JP)
(74) Representative: VOSSIUS & PARTNER
(86) International application number: JP0000010
(87) International publication number: WO0040720

(57) **Abstract**

The present invention relates to a cell that expresses a gene encoding a ligand responsive transcriptional regulatory factor and that comprises the following genes (a) and (b) introduced to the chromosomes thereof:
(a) a reporter gene ligated downstream of a recognition sequence for the ligand responsive transcriptional regulatory factor and a nucleotide sequence necessary for the initiation of transcription; and
(b) a reporter gene that encodes a protein distinguishable from a protein encoded by the above reporter gene and that is ligated downstream of a promoter making no change of transcriptional activity from a ligand contact with the ligand responsive transcriptional regulatory factor, *etc.* According to the present invention, it becomes possible to more conveniently and accurately measure the activity of a test substance against the ability of a ligand responsive transcriptional regulatory factor to promote transcription.

## Description

### BACKGROUND OF THE INVENTION

### Technical Field of the Invention

The present invention relates to cells for measuring the ability to promote transcription. More particularly, it relates to cells with which the activity of a chemical substance against the ability of a ligand responsive transcriptional regulatory factor to promote transcription can be measured.

### Prior Art

The "ligand responsive transcriptional regulatory factor" refers to a protein that can be activated by binding to a ligand, to thereby further bind to a recognition sequence for the ligand responsive transcriptional regulatory factor, which is present in the transcriptional regulatory region for a target gene on the chromosomes, thus exhibiting the function of promoting the transcription of the target gene. This factor plays an important role in organisms in homeostasis maintenance, reproduction, development and growth, cell differentiation, energy metabolism, drug metabolism, and other events in the organisms. It has been known that a failure in the transcriptional regulation of a target gene by such ligand responsive transcriptional regulatory factor leads to an abnormality in the amount of expression of the gene, which may become responsible for various diseases or disorders.

In order to develop a drug useful for the prevention or treatment of such diseases or disorders, or to restrict the intake of a substance responsible for the occurrence of such diseases or disorders, a search has been desired for substances having activity to change the ability of a ligand responsive transcriptional regulatory factor to promote transcription. Many attempts have been made on the development of a method for examining the activity of a chemical substance against the ability of the factor to promote transcription. There has been proposed, for example, a method for examining the activity of a test substance against the ability of a ligand responsive transcriptional regulatory factor to promote transcription, by transiently introducing a reporter gene ligated downstream of a recognition sequence for the ligand responsive transcriptional regulatory factor into cells and then measuring the amount of expression of the reporter gene when these cells are cultured while being brought into contact with the test substance.

In such a method, however, a change in the number of cells by erratic test operations or by the influence of a test substance on the cells may cause the generation of a measurement error in each test run. To obtain a measured value with high accuracy, it requires, in each test run, that the number of cells be counted or that the amount of protein derived from the cells be measured to correct the actually measured value. This makes the operation quite complicated. When a test substance exhibits activity against the transcriptional regulatory system of a gene without mediation of a ligand responsive transcriptional regulatory factor, the actually measured value may fail to accurately reflect the activity of the test substance against the ability of a ligand responsive transcriptional regulatory factor to promote transcription. There has been a desire for the development of a more convenient and accurate method for measuring the activity of a test substance against the ability of a ligand responsive transcriptional regulatory factor to promote transcription.

### SUMMARY OF THE INVENTION

Under these circumstances, the present inventors have conducted extensive studies, and as a result, they have found that the use of cells each having, on the chromosomes thereof, a reporter gene as an index for the ability of a ligand responsive transcriptional regulatory factor to promote transcription and a reporter gene as an index for the number of cells makes it possible to more conveniently and accurately measure the activity of a test substance against the ability of the ligand responsive transcriptional regulatory factor to promote transcription, thereby completing the present invention.

Thus, the present invention provides:
1) A cell that expresses a gene encoding a ligand responsive transcriptional regulatory factor and that comprises the following genes (a) and (b) introduced to the chromosomes thereof (such cell will hereinafter be referred to as "the present cell(s)"):
   (a) a reporter gene ligated downstream of a recognition sequence for the ligand responsive transcriptional regulatory factor and a nucleotide sequence necessary for the initiation of transcription (such reporter gene will hereinafter be referred to as "the ligand responsive reporter gene"); and
   (b) a reporter gene that encodes a protein distinguishable from a protein encoded by the above reporter gene and that is ligated downstream of a promoter making no change of transcriptional activity from a ligand contact with the ligand responsive transcriptional regulatory factor (such reporter gene will hereinafter be referred to as "the cell number reporter gene");
2) A cell that expresses an aryl hydrocarbon receptor gene and that comprises the following genes (a) and (b) introduced to the chromosomes thereof:
   (a) a reporter gene ligated downstream of a recognition sequence for the aryl hydrocarbon receptor and a nucleotide sequence necessary for the initiation of transcription; and
   (b) a reporter gene that encodes a protein distinguishable from a protein encoded by the above reporter gene and that is ligated downstream of a promoter making no change of transcriptional activity from a ligand contact with the ligand responsive transcriptional regulatory factor;
3) A cell that expresses an aryl hydrocarbon receptor gene and that comprises the following genes (a) and (b) introduced to the chromosomes thereof:
   (a) a reporter gene ligated downstream of a recognition sequence for the aryl hydrocarbon receptor and a nucleotide sequence necessary for the initiation of transcription; and
   (b) a reporter gene that encodes a protein distinguishable from a protein encoded by the above reporter gene and that is ligated downstream of a promoter making no change of transcriptional activity from a dioxin contact;
4) A cell that expresses an aryl hydrocarbon receptor gene and an Arnt gene and that comprises the following genes (a) and (b) introduced to the chromosomes thereof:
   (a) a reporter gene ligated downstream of a recognition sequence for the aryl hydrocarbon receptor and a nucleotide sequence necessary for the initiation of transcription; and
   (b) a reporter gene that encodes a protein distinguishable from a protein encoded by the above reporter gene and that is ligated downstream of a promoter making no change of transcriptional activity from a ligand contact with the ligand responsive transcriptional regulatory factor;
5) Use of a cell according to any of the above item 1), 2), 3), or 4) for evaluating the agonist or antagonist activity of a test substance against the ability of a ligand responsive transcriptional regulatory factor to promote transcription in a reporter assay measuring the amount of expression of a reporter gene that undergoes transcriptional regulation by the ligand responsive transcriptional regulatory factor;
6) A method for evaluating the agonist activity of a test substance against the ability of a ligand responsive transcriptional regulatory factor to promote transcription, which comprises the steps of:
   (1) culturing a cell according to any of the above item 1), 2), 3), or 4) in the presence of a test substance, and then measuring the amounts of expression of the reporter genes in this cell;
   (2) selecting a measured value of the amount of expression of the reporter gene ligated downstream of a recognition sequence for the ligand responsive transcriptional regulatory factor and a nucleotide sequence necessary for the initiation of transcription, based on a measured value of the amount of expression of the reporter gene ligated downstream of a promoter making no change of transcriptional activity from a ligand contact; and
   (3) evaluating that the test substance has agonist activity against the ability of the ligand responsive transcriptional regulatory factor to promote transcription, when the measured value of the amount of expression of the reporter gene selected in step (2) is greater than the measured value of the amount of expression of this reporter gene in the absence of the test substance;
7) A method for evaluating the antagonist activity of a test substance against the ability of a ligand responsive transcriptional regulatory factor to promote transcription, which comprises the steps of:
   (1) culturing a cell according to any of the above item 1), 2), 3), or 4) in the presence of a ligand for the ligand responsive transcriptional regulatory factor and a test substance, and then measuring the amounts of expression of the reporter genes in this cell;
   (2) selecting a measured value of the amount of expression of the reporter gene ligated downstream of a recognition sequence for the ligand responsive transcriptional regulatory factor and a nucleotide sequence necessary for the initiation of transcription, based on a measured value of the amount of expression of the reporter gene ligated downstream of a promoter making no change of transcriptional activity from a ligand contact; and
   (3) evaluating that the test substance has antagonist activity. against the ability of the ligand responsive transcriptional regulatory factor to promote transcription, when the measured value of the amount of expression of the reporter gene selected in step (2) is smaller than the measured value of the amount of expression of this reporter gene under the conditions that the ligand is present but the test substance is absent; and
8) A kit for measurement comprising a cell according to any of the above item 1), 2), 3), or 4).

### BRIEF DESCRIPTION OF THE DRAWING

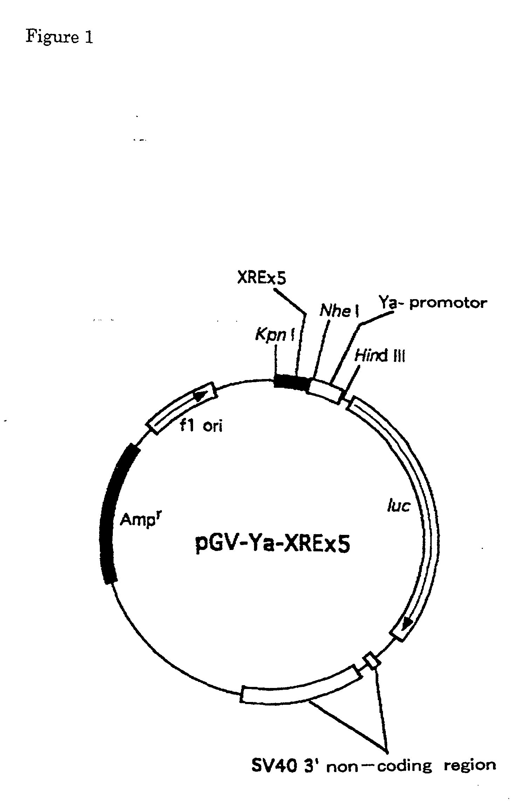

Figure 1 is a map showing the structure of plasmid pGV-Ya-XREx5 containing a reporter gene ligated downstream of a recognition sequence for an aryl hydrocarbon receptor and a nucleotide sequence necessary for the initiation of transcription. In this figure, "luc" refers to the firefly luciferase gene; "Amp"' to the ampicillin resistance gene; "XREx5" to the nucleotide sequence consisting of five repetitions, connected in tandem, of a recognition sequence for an aryl hydrocarbon receptor, which is present in the 5'-upstream region of the rat glutathione S-transferase Ya subunit gene; and "Ya-promoter" to the TATA containing promoter region (*i*.*e*., region consisting of nucleotides -164 to +53 with the transcription initiation site being numbered "1") derived from the 5'-upstream region of the rat glutathione S-transferase Ya subunit gene.

### DETAILED DESCRIPTION OF THE INVENTION

The following will describe the present invention in more detail.

In the present invention, the "ligand responsive transcriptional.regulatory factor" means a receptor-type transcriptional regulatory factor that can be activated by binding to a ligand. Specifically, examples thereof may include nuclear hormone receptors such as steroid hormone receptors (e.g., estrogen receptors, androgen receptors, glucocorticoid receptors), thyroid hormone receptors, fat-soluble vitamin receptors (*e.g.*, vitamin D₃ receptors, retinoic acid receptors) and prostanoid receptors, as well as dioxin receptors (aryl hydrocarbon receptors; hereinafter referred to as "Ah receptor(s)") and insect hormone receptors such as ecdysone receptors.

The present cells can be prepared, for example, by introducing DNA containing the ligand responsive reporter gene and DNA containing the cell number reporter gene into cells each expressing a gene encoding a desired ligand responsive transcriptional regulatory factor, and then screening for the cells each having these genes introduced to the chromosomes thereof.

Examples of the cells which can be used in the preparation of the present cells may include eucaryotic cells such as cells derived from mammal animals (*e*.*g*., humans, mice, rats), cells derived from insect animals, and yeast cells. In view of operability and reproducibility, stably subculturable cells are preferred. More specifically, examples thereof may include human-derived HeLa cells, human-derived MCF7 cells, human-derived HepG2 cells, mouse-derived NIH3T3 cells, mouse-derived Hepalclc7 cells, and rat-derived H4IIE cells [all available from American Type Culture Collection (ATCC)].

Into cells making no production of a desired ligand responsive transcriptional regulatory factor, a gene encoding the desired ligand responsive transcriptional regulatory factor should be introduced so that the gene takes an expressible form. The gene encoding a desired ligand responsive transcriptional regulatory factor may be either a naturally occurring gene or an artificially modified gene, and it may also be, for example, a gene encoding a protein having a ligated functional domain for a different transcriptional regulatory factor. The gene may have the Kozak consensus sequence (Nucleic Acids Res., 12, 857-872 (1984)) ligated upstream of the translation initiation codon ATG. DNA of the gene for the ligand responsive transcriptional regulatory factor can be prepared by designing and producing oligonucleotides for the amplification of DNA encoding the factor, based on the known nucleotide sequences, and then carrying out polymerase chain reaction (hereinafter referred to as "PCR") using the produced oligonucleotides as primers. Examples of DNA which can be used as a template in the PCR may include commercially available cDNA derived from various organisms.

The resulting DNA of the gene for the ligand responsive transcriptional regulatory factor may be, for example, inserted into a vector so that it is ligated in an expressible form to a promoter, and DNA of the resulting vector may be introduced into cells. The promoter should be a promoter functionable in the cells for its introduction, *i.e.*, a promoter having the ability to initiate transcription, and examples may include when the cells are eucaryotic cells, Rous sarcoma virus (RSV) promoter, cytomegarovirus (CMV) promoter, and simian virus (SV40) early or later promoter. The vector may include plasmids each having a replication origin functionable in the microorganisms suitable for gene engineering techniques, such as in *Escherichia coli*, and a drug resistance gene. Specifically, examples thereof may include commercially available vectors for expression, each having a promoter as described above and a gene insertion site downstream thereof.

The DNA containing the gene encoding the ligand responsive transcriptional regulatory factor is introduced into cells as described below to give transformants, preferably stable transformants each having the gene introduced to the chromosomes thereof. The above DNA may be introduced either simultaneously with DNAs containing the reporter genes described below, or separately from such DNAs.

The amount of expression of a desired ligand responsive transcriptional regulatory factor may be increased by introducing a gene encoding the factor into cells expressing a gene encoding the factor so that the gene takes an expressible form. Further, the ability of a ligand responsive transcriptional regulatory factor to promote transcription may be enhanced by introducing into cells and then expressing a gene encoding a protein (*e.g.*, nuclear hormone receptor coactivators) having the function of increasing the amount of transcription of a gene undergoing transcriptional regulation by a ligand responsive transcriptional regulatory factor.

Specifically, for example, when the desired ligand responsive transcriptional regulatory factor is an Ah receptor, the cells which can be used in the preparation of the present cells may be cells expressing an Ah receptor gene, preferably both of this gene and an Arnt [Ah receptor nuclear translocator; Science, 252, 954-958(1991)] gene, and examples may include eucaryotic cells such as cells derived from mammal animals (*e.g.*, humans, mice, rats), cells derived from insect animals, and yeast cells. More specifically, there may be mentioned endogenous Ah receptor gene containing cells such as mouse-derived Hepa1clc7 cells, rat-derived H4IIE cells, and human-derived HepG2 cells. Further, endogenous Ah receptor gene non-containing cells such as yeast cells and CV-1 cells, or cells exhibiting a small amount of expression of an Ah receptor gene, may be used after the amount of expression of the gene is increased by introducing an Ah receptor gene in the cells as described above. As the Ah receptor genes to be introduced into cells, there may be mentioned human-derived Ah receptor genes [GenBank Accession No. L19872; Mol. Pharmacol., 44, 911-917(1993)], mouse-derived Ah receptor genes [GenBank Accession No. M94623; Proc. Natl. Acad. Sci. USA, 89, 8185-8189 (1992)], and rat-derived Ah receptor genes [GenBank Accession No. M94623; Nucleic Acids Res., 22, 3038-3044(1994)].

DNA containing the ligand responsive reporter gene can be prepared, for example, by ligating DNA having a recognition sequence for a desired ligand responsive transcriptional regulatory factor, DNA having a nucleotide sequence necessary for the initiation of transcription, and DNA of the reporter gene so that these pieces of DNA are connected in this order from upstream to downstream.

The reporter gene which can be used in the preparation of the ligand responsive reporter gene may preferably be a gene making it easy to measure the amount of expression, for example, based on the enzyme activity of a protein encoded by the gene, and examples thereof may include genes each encoding firefly luciferase, sea pansy luciferase, β-galactosidase, chloramphenicol acetyltrasferase, or alkaline phosphathase. DNA of the reporter gene can be obtained, for example, by digesting commercially available plasmid DNA containing the reporter gene with a restriction enzyme and then isolating the desired DNA.

The "recognition sequence for a ligand responsive transcriptional regulatory factor" refers to a specific nucleotide sequence present in the transcriptional regulatory region for a target gene, of which amount of expression is regulated by the ligand responsive transcriptional regulatory factor, and when a complex of the ligand responsive transcriptional regulatory factor with a ligand recognizes the sequence and binds thereto, the transcription of the target gene present downstream thereof is promoted. The sequence is usually classified, for example, depending on the kind of the corresponding ligand, into the glucocorticoid responsive element (GRE; Nature, 318, 635-641(1985)), the estrogen responsive element (ERE), and the dioxin responsive element (DRE; J. Biol. Chem., 263, 17221-17224(1988); this may also be referred to as the xenobiotic responsive element (XRE)). Specifically, examples of the recognition sequence for Ah receptors, as the dioxin responsive element, may include nucleotide sequences in the 5'-upstream region of mammal-derived genes, such as cytochrome P450 1A1 gene [cyp1A1; J. Biol. Chem., 263, 17221-17224(1988); Nucleic Acids Res., 15, 4179-4191(1987)], glutathione S-transferase Ya subunit gene [Proc. Natl. Acad. Sci. USA, 87, 3826-3830(1990)], and UDP-glucuronyl transferase gene [J. Biol. Chem., 271, 3952-3958(1996)]. They may also include nucleotide sequences containing one or more repetitions of the consensus sequence [core sequence: 5'-(T/A)GCGTG; J. Biol. Chem., 271, 3952-3958(1996)] for the dioxin responsive element. Examples of the recognition sequence for estrogen receptors, as the estrogen responsive element, may include a nucleotide sequence in the 5'-upstream region of the vitellogenin gene from *Xenopus laevis* (Cell, 57, 1139-1146). They may also include nucleotide sequences containing one or more repetitions of the consensus sequence [5'-AGGTCAnnnTGACCTT-3'] for the estrogen responsive element. To obtain an ample ability to promote transcription, it is usually preferred that the consensus sequence as described above is repeatedly connected about two to five times in tandem. DNA having such nucleotide sequence can be prepared, for example, by chemical synthesis or by PCR amplification and cloning.

The "nucleotide sequence necessary for the initiation of transcription" refers to a nucleotide sequence having a TATA box and a leader sequence for the initiation of transcription, and examples thereof may include a nucleotide sequence in the 5'-upstream region of the thymidine kinase (tk) gene, a nucleotide sequence in the 5'-upstream region of the glutathione S-transferase Ya subunit gene (*e.g.*, region containing nucleotides -164 to +66 with the transcription initiation site being numbered as "1"; Proc. Natl. Acad. Sci. USA, 87, 3826-3830(1990)), and a nucleotide sequence in the 5'-upstream region of the cytochrome P450 1A1 gene (*e.g.*, region containing nucleotides -70 to +120 with the transcription initiation site being numbered "1"; Eur. J. Biochem., 159, 219-225(1986)). DNA having such nucleotide sequence can be prepared, for example, by designing and producing oligonucleotides for the amplification of DNA encoding a region as described above, based on the known nucleotide sequences, and carrying out PCR using the produced oligonucleotides as primers.

DNA containing the cell number reporter gene can be prepared, for example, by ligating DNA of a promoter making no change of transcriptional activity from the ligand contact with the desired ligand responsive transcriptional regulatory factor, and DNA of the reporter gene.

The reporter gene which can be used for the cell number reporter gene may preferably be a gene making it easy to measure the amount of expression, for example, by the enzyme activity of a protein encoded by the gene, and a different reporter gene is used, encoding a protein distinguishable from the protein encoded by the reporter gene used for the above ligand responsive reporter gene. Distinction may be carried out, for example, by a difference of enzyme activity or substrate specificity. DNA of the reporter gene can be obtained, for example, by digesting commercially available plasmid DNA containing the reporter gene with a restriction enzyme and then isolating the desired DNA.

The "promoter making no change of transcriptional activity from the ligand contact with the ligand responsive transcriptional regulatory factor" refers to a promoter having the constitutive ability to make transcription without undergoing regulation by the recognition sequence for the ligand responsive transcriptional regulatory factor as described above, and examples thereof may include tk promoter, RSV promoter, and CMV promoter. DNA of the promoter can be obtained, for example, by digesting commercially available plasmid DNA containing the promoter with a restriction enzyme and then isolating the desired DNA.

DNA containing the cell number reporter gene can also be prepared by inserting DNA of the reporter gene downstream of the promoter in a commercially available plasmid containing the promoter.

The present cells can be prepared by incorporating into vectors such as plasmids, DNA containing the ligand responsive reporter gene and DNA containing the cell number reporter gene, respectively, and introducing these vectors into the above cells, and then screening the cells each having these reporter genes introduced to the chromosomes thereof. Specifically, for example, when the desired ligand responsive transcriptional regulatory factor is an Ah receptor, a plasmid having the firefly luciferase gene (ligand responsive reporter gene) ligated downstream of a nucleotide sequence containing the dioxin responsive element derived from the 5'-upstream region of the cytocrome P450 1A1 gene and a nucleotide sequence necessary for the initiation of transcription derived from the 5'-upstream region of the glutathione S-transferase Ya subunit gene, and a plasmid having the sea pansy luciferase gene (cell number reporter gene) ligated downstream of the tk promoter are produced, and these plasmids are introduced into endogenous Ah receptor gene containing Hepalclc7 cells. In this case, to make easy screening of cells having these reporter genes introduced, it may involve cointroduction of plasmids for expression of screening marker genes such as drug resistance genes. Examples of the drug resistance gene which can be used in such a manner may include neomycin resistance (aminoglycoside phosphotransferase) gene, blasticidin S resistance gene, and hygromycin resistance gene.

To introduce DNA of a plasmid having both the above reporter genes, for example, into the cells derived from mammal animals, for example, the cells are first plated on a culture vessel (10⁵ to 10⁷ cells/petri dish of 6 to 10 cm in diameter), followed by culturing for about several hours to overnight at 37°C under 5% CO₂ and saturated humidity conditions, using αMEM medium containing about 5% to 10% (v/v) serum. Into the cells thus cultured, plasmid DNA having the reporter genes is introduced. As the method for DNA introduction into cells, there may be mentioned ordinary lipofection methods, DEAE-dextran methods, calcium phosphate methods, and electroporation methods. Specifically, for example, when commercially available lipofectin (available from GIBCO-BRL) is used, an operation may be carried out according to the manual attached thereto, and regarding the amount of plasmid DNA introduced, the amount of lipofection, the kind of cells, the number of cells, and other parameters, optimum conditions may have previously been determined by a preliminary study. When a plasmid containing a drug resistance gene is cointroduced with plasmids having the reporter genes, it may be preferred to set the amount of plasmid DNA containing the drug resistance gene about 1/5 to 1/10 smaller than the amount of plasmid DNA having the reporter genes. For the plasmid DNA, there may be used plasmid DNA linearized by previously digesting DNA, which has been purified by the CsCl density gradient centrifugation method, or DNA of similar purity, with a restriction enzyme having no recognition sites in the regions necessary for the preparation of the present cells (*e.g.*, recognition sequence for the ligand responsive transcriptional regulatory factor, promoter, reporter genes, screening marker gene). After the introduction of plasmid DNA into cells, the medium is replaced with a serum containing medium, and culturing is continued overnight to for about 2 days. The cells are then scratched from the culture vessel, for example, by trypsin treatment according to the ordinary method, and plated in a new culture vessel. Immediately after the plating, or after further culturing for 1 or 2 days, the medium is replaced with a conditioned medium corresponding to the screening marker gene introduced into the cells, and culturing in the conditioned medium corresponding to the screening marker gene is continued until non-transformed cells are dead and colonies derived from transformed cells become an appropriate size. During this period, if necessary, medium replacement is made at a rate of 1 to 2 times/week. These operations provide the incorporation of the introduced reporter genes to the chromosomes in the cells, and provide the cells stably holding the reporter genes therein. If necessary, the operation for the introduction of reporter genes as described above may be repeated.

To verify that the introduced reporter genes have been incorporated to the chromosomes in the cells, genomic DNA of the cells may be prepared according to the ordinary gene engineering method and then detected for the presence of the reporter genes by making use of PCR, southern hybridization, or other methods with DNA fragments having partial nucleotide sequences of the introduced reporter genes as primers or probes.

Using the ligand response activity of the amounts of expression of the reporter genes in the cells thus obtained as an index, preferred cells can be selected for measuring the agonist or antagonist activity of a test substance against the ability of a ligand responsive transcriptional regulatory factor to promote transcription. Specifically, the colonies thus obtained are first divided into plural groups and re-plated, and the cells are grown, after which a solution of a ligand for the desired ligand responsive transcriptional regulatory factor is added to one portion thereof (*i.e.*, ligand added group), followed by culturing for about 4 hours to 2 days and then measuring the amounts of expression of the ligand responsive reporter gene and the cell number reporter gene. In addition, as a control, only a solvent used in the preparation of the above ligand solution is added to another portion of the above cells (*i.e.*, control group), followed by culturing and then measuring the amounts of expression of the respective reporter genes in the same manner.

The method for measuring the amounts of expression of the reporter genes, although it depends on the kinds of reporter genes used, may usually involve adding a cell lysis buffer to the cells subject to measurement, thereby preparing a cell extract, and then measuring the amounts of proteins encoded by the reporter genes, which are contained in the resulting extracts. For example, when the proteins encoded by the reporter genes have enzyme activity, a cell extract containing proteins encoded by the reporter genes is reacted with substrates specific to the enzymes, and the amounts of remaining substrate or the amounts of reaction products are measured with their amounts of luminescence, fluorescent absorbance, or absorbance as an index. Specifically, for example, when luciferase genes are used as the reporter genes, the reaction of a cell extract with luciferin as the substrate of luciferases produces luminescence at a strength in proportion to the amounts of luciferases in the cell extracts. Therefore, the amounts of luciferases in the cell extract as well as the amounts of expression of luciferase genes can be known by measuring the strength of luminescence with a measuring device such as a luminometer.

For the amount of expression of each reporter gene, an increase in the amount of expression of each reporter gene from said ligand contact is achieved by subtracting the amount of expression in the control group from the amount of expression in the ligand added group. Then, cells are selected, exhibiting an increase in the amount of expression of the ligand responsive reporter gene from said ligand contact, which is at least 2 times greater, preferably at least 10 times greater, than the amount of expression in the control group. Further, in the present cells, because it is preferred that the amount of expression of the cell number reporter gene makes no change from a ligand contact, cells may be selected, exhibiting an increase in the amount of expression of the cell number reporter gene from said ligand contact, which is at least half of or lower than, an increase in the amount of expression of the ligand responsive reporter gene from the same ligand contact. In this case, when the colonies thus obtained are not formed by identical transformed cells, these cells are diluted and further cultured, and colonies formed by identical cells are selected.

Using the present cells thus obtained, the agonist or antagonist activity of a test substance against the ability of a ligand responsive transcriptional regulatory factor to promote transcription can be measured.

For example, the present cells are plated on 96-well plates at a ratio of about 10³ to 10⁴ per well, and 5% to 10% (v/v) serum containing medium (*e*.*g*, αMEM medium) was added to the wells in 100 to 200 µl, followed by culturing at 37°C under 5% CO₂ and saturated humidity conditions for about several hours to overnight. A solution of a test substance dissolved in a solvent, or only a solvent is added so that the final concentration comes to 0.5% (v/v) to 2% (v/v) or lower. For the solvent, dimethylsulfoxide (DMSO), ethanol, or other solvents can be used, and the use of DMSO is desired from the viewpoint of its small influence on the cells. When a test substance is an aqueous solution, it is sterilized by filtration through a filter of 20 µm in pore size and then added to the above culture system so that the final concentration comes to 10% (v/v) or lower. After the addition of a test substance, culturing is continued for about 4 hours to 2 days, and the amounts of expression of the ligand responsive reporter gene and the cell number reporter gene are measured in such a manner as described above.

For the measured values of the amount of expression of the cell number reporter gene, a ratio is determined of the measured value in the test substance added group to the measured value in the only solvent added group (control group). When the ratio is, for example, in the range of 0.8 to 1.2, the measured value of the amount of expression of the ligand responsive reporter gene in the test substance added group is selected for evaluating the agonist or antagonist activity of the test substance against the ability of a ligand responsive transcriptional regulatory factor to promote transcription. When the ratio takes a small value such as smaller than 0.8, for example, it is possible that the test substance has cytotoxicity. In contrast, when the ratio takes a large value such as greater than 1.2, for example, it is possible that the test substance has activity against the transcriptional regulatory system of a gene without mediation of a ligand responsive transcriptional regulatory factor. However, the range of this ratio suitable for selecting the measured value of the amount of expression of the reporter responsive reporter gene may vary depending on certain properties of the present cells used and the conditions of measurement, and it is not limited to the above examples of numerical values.

When the thus selected measured value of the amount of expression of the ligand responsive reporter gene in the test substance added group is greater than the measured value in the only solvent added group (control group), the test substance can be evaluated to have agonist activity against the ability of a ligand responsive transcriptional regulatory factor, which is subject to measurement by the present cells, to promote transcription. In this case, the selected measured value of the amount of expression of the ligand responsive reporter gene may be corrected based on the measured value of the amount of expression of the cell number reporter gene in the test group, and the activity of the test substance may be evaluated based on the resulting corrected value.

Alternatively, the present cells are cultured, for example, in the presence of a ligand for the ligand responsive transcriptional regulatory factor subject to measurement, or in the presence of the ligand and a test substance, and the amounts of expression of the ligand responsive reporter gene and the cell number reporter gene in each case are measured in the same manner as described above. For the measured values of the amount of expression of the cell number reporter gene, a ratio is determined of the measured value in the ligand and test substance added group to the measured value in the only ligand added group. When the ratio is, for example, in the range of 0.8 to 1.2, the measured value of the amount of expression of the ligand responsive reporter gene in the test substance added group is selected for evaluating the activity of the test substance against the ability of a ligand responsive transcriptional regulatory factor to promote transcription. When the thus selected measured value of the amount of expression of the ligand responsive reporter gene in the ligand and test substance added group is smaller than the measured value in the only ligand added group (control group), the test substance can be evaluated to have antagonist activity against the ability of a ligand responsive transcriptional regulatory factor, which is subject to measurement by the present cells, to promote transcription. In this case, the selected measured value of the amount of expression of the ligand responsive reporter gene may be corrected based on the measured value of the amount of expression of the cell number reporter gene in the test group, and the antagonist activity of the test substance may be evaluated based on the resulting corrected value.

By the use of the present cells, the amount of expression of the ligand responsive reporter gene and the amount of expression of the cell number reporter gene can be measured using the same cell extract, so that a change in the number of cells by erratic test operations or by the influence of a test substance on the cells can be known without need of a complicated operation. Accordingly, not only appropriate measured values can be selected, but also actually measured values can easily be corrected, thereby making it possible to more conveniently and accurately measure the activity of a test substance against the ability of a ligand responsive transcriptional regulatory factor to promote transcription. In addition, the present cells can be freeze-stored, and when needed, they can be used by awakening, so that the preparation work for the present cells (*e*.*g*., reporter gene introducing operation, cell screening operation), which would otherwise be needed in each test run, can be omitted, and measurement can be carried out with high reproducibility using the cells with consistent performance.

The present invention will hereinafter be further illustrated by some Examples and Test Examples; however, the present invention is not limited to these Examples.

### Example 1: Construction of ligand responsive reporter gene containing plasmid

Using rat genomic DNA as a template and using an oligonucleotide of the nucleotide sequence: 5'-gcgctagcatggtagcgccttgtcagcc-3' (SEQ ID NO:1) and an oligonucleotide of the nucleotide sequence: 5'-gcaagcttgagtactgacctagcgagag-3' (SEQ ID NO:2) as primers, PCR was carried out to amplify DNA containing a promoter region derived from the 5'-upstream region of the rat glutathione S-transferase Ya subunit gene (*i*.*e*., region consisting of nucleotides -164 to +53 with the transcription initiation site being numbered "1") (Proc. Natl. Acad. Sci. USA, 87, 3826-3830 (1990)), followed by cloning. The above DNA cloned was inserted between the *Hind* III site and the *Nhe* I site of reporter plasmid pGV-P (marketed by Toyo Ink; containing the firefly luciferase gene) to give plasmid pGV-Ya.

Separately, two oligonucleotides as described below, each nucleotide sequence containing a recognition sequence for an aryl hydrocarbon receptor, which is present in the 5'-upstream region of the rat glutathione S-transferase Ya subunit gene (*i.e.*, dioxin responsive element; Proc. Natl. Acad. Sci. USA, 87, 3826-3830 (1990)) were synthesized.

Oligonucleotide A: 5'-ctcaggcatgttgcgtgcatccctgaggccagccgagct-3'
(SEQ ID NO:3)

Oligonucleotide B: 5'-cggctggcctcagggatgcacgcaacatgcctgagagct-3'
(SEQ ID NO:4)

The above oligonucleotides A and B were then independently treated with T4 polynucleotide kinase to provide 5'-end phosphorylation, followed by annealing, to give double-strand DNA. The double-strand DNA was purified by agarose gel electrophoresis, followed by ligation reaction, to give DNA having five repetitions, connected in tandem, of the double-strand DNA (such DNA will hereinafter be referred to as "XREx5"). The DNA XREx5 was inserted into the Sac I digestion site of plasmid pGV-Ya described above to give reporter gene containing plasmid pGV-Ya-XREx5 (ligand responsive reporter gene containing plasmid; Figure 1) having the firefly luciferase gene ligated downstream of a recognition sequence for an aryl hydrocarbon receptor (*i.e.*, dioxin responsive element) and a nucleotide sequence necessary for the initiation of transcription.

### Example 2: Preparation of cell number reporter gene containing plasmid and production I of the present cells

Hepalclc7 cells (obtained from ATCC) were plated on two petri dishes of 10 cm in diameter at a ratio of 5 x 10⁵ cells per dish and cultured overnight in αMEM medium supplemented with 10% (v/v) fetal bovine serum (such medium will hereinafter be referred to as "10% serum containing αMEM medium") at 37°C under 5% CO₂. In this Example, all the following culturing was carried out at 37°C under 5% CO₂.

Separately, plasmid pGV-Ya-XREx5 (ligand responsive reporter gene containing plasmid) constructed in Example 1 was digested with *Bam* HI. Further, plasmid pRL-TK (cell number reporter gene containing plasmid; marketed by Toyo Ink) containing the sea pansy luciferase gene used as the cell number reporter gene was digested with *Bam* HI, and neomycin resistance (aminoglycoside phosphotransferase) gene containing plasmid pRc/RSV (available from INVITROGEN) was digested with *Hind* III. These three digested plasmids, *i.e., Bam* HI digested pGV-Ya-XREx5 (2.8 µg), *Bam* HI digested pRL-TK (2.8 µg), and *Hind* III digested pRc/RSV (0.5 µg), were then mixed with 300 µl of αMEM medium (mixture A), while 36 µl of lipofectin (available from GIBCO-BRL) was mixed with 300 µl of αMEM medium (mixture B). After being left at room temperature for 30 minutes, mixtures A and B were combined together and left at room temperature for 10 minutes. The Hepa1clc7 cells cultured overnight as described above were then washed once with αMEM medium, to which there was added a solution prepared by adding and mixing additional 5.4 ml of αMEM medium to the above mixture (A + B). Culturing of the resulting mixture was carried out for 5 hours. The medium was then replaced with 10% serum containing αMEM medium, followed by further culturing overnight. Subsequently, the cells were scratched from the petri dishes and collected, and the collected cells were suspended in 10% serum containing αMEM medium to have the total volume of 100 ml. The resulting cell suspension was plated on 96 well plates at a ratio of 100 µl/well. After these plates were cultured overnight, a medium prepared by dissolving geneticin (hereinafter referred to as "G418") in 10% serum containing αMEM medium to have a concentration of 1.6 mg/ml was added at a ratio of 100 µl/well (G418 final concentration 800 µg/ml). Culturing was further continued for 3 weeks, during which the medium was replaced with 10% serum containing αMEM medium containing 800 µg/ml G418 at a rate of once per week. The volume of the medium was set at 200 µl/well. After said 3 weeks, cells were scratched from the wells in which colony formation had been observed, and collected (for 192 wells), and the collected cells were divided into three and independently plated on different plates, followed by continuous culturing for 4 days. To the first plate, only DMSO was added (solvent control), and to the second plate, 3-methylcholanthrene (hereinafter referred to as "3-MC") dissolved in DMSO was added so that the final concentration of 3-MC in the second plate came to 10 µM per well. The first and second plates were then cultured overnight. The third plate was further cultured as the master plate without any addition. From each well of each plate, to which DMSO or 3-MC had been added, the medium was removed, and the wells were washed twice with PBS(-) (sodium chloride, 8000 mg/l; potassium chloride, 200 mg/l; sodium monohydrogenphosphate (anhydrous), 1150 mg/l; sodium dihydrogenphosphate (anhydrous), 200 mg/l; marketed by Nissui Pharmaceutical). A 5-fold diluted cell lysis buffer of PicaGene dual kit (marketed by Toyo Ink) was added to the wells in 15 µl/well, followed by leaving the plates at room temperature for 30 minutes or longer to cause cell lysis, to give cell extracts. These plates were set on a luminometer LB96P with an automatic enzyme substrate injector (available from Berthold), and PicaGene luminescence reagent II and sea pansy luminescence reagent (PicaGene dual kit, marketed by Toyo Ink) were automatically dispensed each in 50 µl, followed by continuous measurement for the amount of luminescence. Six clones were selected from those exhibiting a higher increase in the amount of expression of the firefly luciferase gene (ligand responsive reporter gene) by the addition of 3-MC, and the corresponding clone cells were separated from the master plate and plated. The resulting cells were used to carry out the luciferase assay in the same manner, and four clones were selected, exhibiting an at least two times higher increase in the expression of the firefly luciferase gene by the addition of 3-MC. Further, for four clones thus selected, the same cell plating and luciferase assay as described above were carried out, and a clone was selected, exhibiting an about 10 times higher increase in the expression of the firefly luciferase gene by the addition of 3-MC.

### Example 3: Preparation II of the present cells

1 x 10⁶ Hepa1clc7 cells (ATCC) were plated on petri dishes of 10 cm in diameter and cultured overnight in 10% serum containing αMEM medium at 37°C under 5% CO₂ in the same manner as described in Example 2. In this Example, all the following culturing was carried out at 37°C under 5% CO₂.

Separately, the ligand responsive reporter gene containing plasmid pGV-Ya-XREx5 constructed in Example 1 was digested with *Bam* HI. Further, the cell number reporter gene containing plasmid pRL-TK (marketed by Toyo Ink) was digested with *Bam* HI, and the neomycin resistance (aminoglycoside phosphotransferase) gene containing plasmid pRc/RSV (available from INVITROGEN) was digested with *Hind* III. These three digested plasmids, *i.e.*, *Bam* HI digested pGV-Ya-XREx5 (5.6 µg), *Bam* HI digested pRL-TK (5.6 µg), and *Hind* III digested pRc/RSV (1 µg), were mixed with 600 µl of αMEM medium (mixture C), while 72 µl of lipofectin (available from GIBCO-BRL) was mixed with 600 µl of αMEM medium (mixture D). After being left at room temperature for 30 minutes, mixtures C and D were combined together and left at room temperature for 10 minutes. After the Hepalclc7 cells cultured overnight as described above were then washed once with αMEM medium, there was added a half volume of a solution prepared by adding and mixing additional 10.8 ml of αMEM medium into the above mixture (C + D). Culturing of the resulting mixture was carried out for 5 hours. The medium was then replaced with 10% serum containing αMEM medium, followed by further culturing for 2 days. Subsequently, the cells were scratched from the petri dishes and collected, and the collected cells were suspended in 10% serum containing αMEM medium to have the total volume of 100 ml. The resulting cell suspension was plated on 96-well plates at a ratio of 100 µl/well. After these plates were cultured overnight, a medium prepared by dissolving G418 in 10% serum containing αMEM medium to have a concentration of 3.2 mg/ml was added at a ratio of 100 µl/well (G418 final concentration 1.6 mg/ml). Culturing was further continued, during which the medium was replaced with 10% serum containing αMEM medium containing 800 µg/ml G418 at a rate of once per 4 to 5 days. The volume of the medium was set at 200 µl/well. After culturing for 15 days, colonies were observed in almost all the wells, and cells were scratched from four plates and collected (for 384 wells), and the collected cells were divided in three and independently plated on different plates, followed by further culturing for 1 day. To the first plate, only DMSO was added (solvent control), and to the second plate, 3-MC dissolved in DMSO was added so that the final concentration of 3-MC in the second plate came to 2 µm per well. The first and second plates were then cultured overnight. In this case, the DMSO or the solution of 3-MC in DMSO was added so that the final concentration of DMSO in each well came to 0.04%. The third plate was further cultured as the master plate. From each well of each plate, to which DMSO or 3-MC had been added, the medium was removed, and the wells were washed twice with PBS(-). A 5-fold diluted cell lysis buffer was added to the wells in 20 µl/well, followed by leaving the plates at room temperature for 30 minutes or longer to cause cell lysis, to give cell extracts. The resulting cell extracts were transferred in 10 µl to white 96-well plates. These plates were set on a luminometer LB96P with an automatic enzyme substrate injector (available from Berthold), and PicaGene luminescence reagent (sea pansy luciferase substrate solution PGL100, marketed by Toyo Ink) was automatically dispensed each in 50 µl, followed by measurement for the amount of luminescence. Four clones were selected, exhibiting a high increase in the amount of expression of the sea pansy luciferase gene (ligand responsive reporter gene) and a high measured value of the amount of luminescence by the addition of 3-MC. For these four clones, the corresponding clone cells were grown from the master plate and then plated on two 96-well plates to have a ratio of about 1 cell per well, followed by continuous culturing in 10% serum containing αMEM medium which comprises 800 µg/ml G418, during which the medium was replaced once in about 5 days. After 2 weeks, for each of the above four clones, 12 colonies produced from identical cells (identical colonies) were selected and divided into three as described above. Two of them were plated on 96-well plates for luciferase luminescence measurement and for culturing (available from Corning Coster; #3903), and the other one was transferred to transparent 48-well plates, followed by culturing for 1 day. To the first plate, only DMSO was added (solvent control), and to the second plate, 3-MC was added to have a final concentration of 2 µM per well. The first and second plates were then cultured overnight. From each well of each plate, to which DMSO or 3-MC had been added, the medium was removed, and the wells were washed twice with PBS(-). A 5-fold diluted cell lysis buffer was added to the wells in 15 µl/well, followed by leaving the plates at room temperature for about 1 hour to cause cell lysis, to give cell extracts. The resulting cell extract containing plates were set on a luminometer LB96P with an automatic enzyme substrate injector (available from Berthold), and PicaGene luminescence reagent II and sea pansy luminescence reagent (PicaGene dual kit, marketed by Toyo Ink) were automatically dispensed each in 50 µl, followed by continuous measurement for the amount of luminescence. All the clones exhibited quite small activity of sea pansy luciferase, and it was therefore considered that no sea pansy luciferase gene had been introduced. For one clone exhibiting a good increase in the amount of expression of the firefly luciferase gene (ligand responsive reporter gene) by the addition of 3-MC, the sea pansy luciferase gene was introduced once again. As such, the clone cells were plated on petri dishes of 10 cm in diameter to have 1 x 10⁶ cells per dish and cultured overnight in 10% serum containing αMEM medium. Separately, plasmid pRL-TK was digested with *Bam* HI, and plasmid pUC-BSD (marketed by Kaken Pharmaceutical; containing blasticidin S (hereinafter referred to as "BSD") resistance gene) was digested with *Eco.* RI. These two digested plasmids, *i.e.*, *Bam* HI digested pRL-TK (2 µg) and *Eco* RI digested pUC-BSD (0.5 µg) were mixed with 300 µl of αMEM medium (mixture E), while 36 µl of lipofectin (available from GIBCO-BRL) was mixed with 300 µl of αMEM medium (mixture F). After being left at room temperature for about 40 minutes, mixtures E and F were combined together and left at room temperature for about 15 minutes. After the Hepalclc7 cells, which had been overnight cultured as described above, were washed once with αMEM medium, there was added a solution prepared by adding and mixing additional 4.4 ml of αMEM medium to the above mixture (E + F). Culturing of the resulting mixtures was carried out for 6 hours. The medium was replaced with 10% serum containing αMEM medium containing 100 international units/ml (final concentration) penicillin and 100 µg/ml (final concentration) streptomycin (hereinafter referred to as "P-S serum containing αMEM medium"), followed by culturing overnight. Subsequently, the cells were scratched from the plates and collected, and the collected cells were suspended in P-S serum containing αMEM medium to have the total volume of 100 ml. The resulting cell suspension was plated on ten 96-well plates at a ratio of 100 µl/well, followed by culturing overnight. After medium removal, 10% serum containing αMEM medium, in which BSD and G418 had been dissolved to have a concentration of 16 µg/ml and 800 µg/ml, respectively, was added to each well at a ratio of 200 µl/well, and culturing was continued, while making medium replacement once in four or five days. After about 2 weeks, cells were scratched from the wells in which colonies had been observed (72 wells) and collected, and the collected cells were divided in three, of which two were plated on 96-well plates for luciferase luminescence measurement and for culturing (available from Corning Coster; #3903) and the other one was plated on transparent 24-well plates, followed by culturing overnight. To the first plate, only DMSO was added (solvent control), and to the second plate, 3-MC was added to have a final concentration of 2 µM per well. The first and second plates were then cultured for 6.5 hours. The third plate was further cultured as the master plate. From each well of each plate, to which DMSO or 3-MC had been added, the medium was removed, and the wells were washed twice with PBS(-). A 5-fold diluted cell lysis buffer was added in 15 µl/well, followed by leaving the plates at room temperature for 1 hour or longer to cause cell lysis, to give cell extracts. The resulting cell extract containing plates were set on a luminometer LB96P with an automatic enzyme substrate injector (available from Berthold), and PicaGene luminescence reagent II and sea pansy luminescence reagent (PicaGene dual kit, marketed by Toyo Ink) were automatically dispensed in 50 µl, followed by continuous measurement for the amount of luminescence. Five clones were selected, exhibiting a high increase in the amount of expression of the firefly luciferase gene (ligand responsive reporter gene) by the addition of 3-MC, almost no difference in the measured value of the amount of luminescence by sea pansy luciferase between the DMSO added wells and the 3-MC added wells, and high measured values of the respective amounts of luminescence by sea pansy luciferase. For two of these clones, the corresponding clone cells were separated from the master plate and then plated to carry out the luciferase assay in the same manner. One clone was selected, exhibiting approximately the same tendency in the results of measurement for the amount of luminescence by both luciferases as described above.

### Example 4: Use of the present cells in the reporter assay (No. 1)

The clone screened in Example 2 was plated on 96-well plates at a ratio of 40,000 cells/well, followed by culturing overnight. A 50 µg/ml solution of 2,3,7,8-tetrachlorodibenzo-p-dioxin (hereinafter referred to as "TCDD") in nonane (available from Kanto Kagaku) was diluted with DMSO, and the resulting solution was added to the above cells, followed by continuous culturing for about 40 hours. After medium removal, the wells were washed twice with PBS(-). A cell lysis buffer was added to the wells in 20 µl/well, followed by leaving the plates at room temperature for 30 minutes or longer to cause cell lysis, and these plates were freeze-stored at -80°C. These freeze-stored plates were left at room temperature, and melted cell extracts were transferred in 10 µl to white 96-well plates. These plates were set on a luminometer LB96P with an automatic enzyme substrate injector (available from Berthold), and PicaGene luminescence reagent II and sea pansy luminescence reagent (PicaGene dual kit, marketed by Toyo Ink) were automatically dispensed each in 50 µl, followed by continuous measurement for the amount of luminescence. The results are shown in Table 1.

**TABLE 1**

| TCDD final conc. (pg/ml) | Amount of luminescence by firefly luciferase (from pGV-Ya-XREx5) | | Amount of luminescence by sea pansy luciferase (from pRL-TK) | |
|---|---|---|---|---|
| | Measured value (x 10⁴) | Ratio | Measured value (x 10⁶) | Ratio |
| 0 | 0.486 | 1.0 | 1.17 | 1.0 |
| 0.02 | 0.517 | 1.1 | 1.15 | 1.0 |
| 0.2 | 0.532 | 1.1 | 1.10 | 0.9 |
| 2 | 0.873 | 1.8 | 1.08 | 0.9 |
| 20 | 3.14 | 6.5 | 0.894 | 0.8 |
| 200 | 4.46 | 9.2 | 0.783 | 0.7 |
| 2000 | 4.17 | 8.6 | 0.691 | 0.6 |
| 20000 | 4.49 | 9.2 | 0.728 | 0.6 |

### Example 5: Use of the present cells in the reporter assay (No. 2)

The clone screened in Example 3 was plated on 96-well plates at a ratio of 5000 cells/100 µl medium/well, followed by culturing overnight. Then, 3-MC and benzo[a]pyrene, as typical Ah receptor activating compounds, and dexamethasone, as an Ah receptor non-activating compound, were independently dissolved in DMSO, and the resulting solutions was independently added to the above cultured cells so that the final concentration of each compound in the medium came to 1, 10, 10², 10³, 10⁴, or 5 x 10⁴ nM. In this case, these solutions were prepared and added so that the final concentration of DMSO in the medium came to 0.25% (v/v). After about 6 hours from the compound addition, the medium was removed, and the wells were washed twice with PBS(-). A 5-fold diluted cell lysis buffer was added to the wells in 15 µl/well, followed by leaving the plates at room temperature for about 1 hour. The resulting cell extract containing plates were freeze-stored at -80°C. These freeze-stored plates were left at room temperature, and the melted cell extract containing plates were set on a luminometer LB96P with an automatic enzyme substrate injector (available from Berthold), and PicaGene luminescence reagent II and sea pansy luminescence reagent (PicaGene dual kit, marketed by Toyo Ink) were automatically dispensed each in 50 µl, followed by continuous measurement for the amount of luminescence. The results are shown in Tables 2 to 4.

**TABLE 2**

| Dexamethasone final conc. (nM) | Amount of luminescence by firefly luciferase (from pGV-Ya-XREx5) | | Amount of luminescence by sea pansy luciferase (from pRL-TK) | |
|---|---|---|---|---|
| | Measured value (x 10²) | Ratio | Measured value (x 10⁴) | Ratio |
| 0 | 2.87 | 1.0 | 3.95 | 1.0 |
| 1 | 3.71 | 1.3 | 3.89 | 1.0 |
| 10 | 3.54 | 1.2 | 3.85 | 1.0 |
| 100 | 3.39 | 1.2 | 3.40 | 0.9 |
| 1000 | 3.22 | 1.1 | 3.24 | 0.8 |
| 10000 | 3.53 | 1.2 | 3.97 | 1.0 |
| 50000 | 3.73 | 1.3 | 4.24 | 1.1 |

**TABLE 3**

| Benzo[a]pyrene final conc. (nM) | Amount of luminescence by firefly luciferase (from pGV-Ya-XREx5) | | Amount of luminescence by sea pansy luciferase (from pRL-TK) | |
|---|---|---|---|---|
| | Measured value (x 10²) | Ratio | Measured value (x 10⁴) | Ratio |
| 0 | 0.251 | 1.0 | 2.94 | 1.0 |
| 1 | 0.414 | 1.7 | 2.65 | 0.9 |
| 10 | 0.928 | 3.7 | 2.76 | 0.9 |
| 100 | 1.72 | 6.9 | 2.44 | 0.8 |
| 1000 | 3.12 | 12 | 2.56 | 0.9 |
| 10000 | 4.54 | 18 | 2.44 | 0.8 |
| 50000 | 5.76 | 23 | 2.21 | 0.8 |

**TABLE 4**

| 3-MC final conc. (nM) | Amount of luminescence by firefly luciferase (from pGV-Ya-XREx5) | | Amount of luminescence by sea pansy luciferase (from pRL-TK) | |
|---|---|---|---|---|
| | Measured value (x 10³) | Ratio | Measured value (x 10⁴) | Ratio |
| 0 | 0.374 | 1.0 | 4.81 | 1.0 |
| 1 | 1.17 | 3.1 | 4.48 | 0.9 |
| 10 | 2.19 | 5.9 | 4.62 | 1.0 |
| 100 | 3.83 | 10 | 4.33 | 0.9 |
| 1000 | 4.89 | 13 | 4.17 | 0.9 |
| 10000 | 8.52 | 23 | 4.03 | 0.8 |
| 50000 | 8.68 | 23 | 4.10 | 0.9 |

### EFFECTS OF THE INVENTION

According to the present invention, it becomes possible to more conveniently and accurately measure the activity of a test substance against the ability of a ligand responsive transcriptional regulatory factor to promote transcription.

### SEQUENCE LISTING FREE TEXT

### SEQ ID NO: 1

Designed oligonucleotide primer for PCR

### SEQ ID NO: 2

Designed oligonucleotide primer for PCR

### SEQ ID NO: 3

Designed oligonucleotides to synthesize DNA containing dioxin responsive element

### SEQ ID NO: 4

Designed oligonucleotides to synthesize DNA containing dioxin responsive element

## Claims

1. A cell that expresses a gene encoding a ligand responsive transcriptional regulatory factor and that comprises the following genes (a) and (b) introduced to the chromosomes thereof:
(a) a reporter gene ligated downstream of a recognition sequence for the ligand responsive transcriptional regulatory factor and a nucleotide sequence necessary for the initiation of transcription; and
(b) a reporter gene that encodes a protein distinguishable from a protein encoded by the above reporter gene and that is ligated downstream of a promoter making no change of transcriptional activity from a ligand contact with the ligand responsive transcriptional regulatory factor.

2. A cell that expresses an aryl hydrocarbon receptor gene and that comprises the following genes (a) and (b) introduced to the chromosomes thereof:
(a) a reporter gene ligated downstream of a recognition sequence for the aryl hydrocarbon receptor and a nucleotide sequence necessary for the initiation of transcription; and
(b) a reporter gene that encodes a protein distinguishable from a protein encoded by the above reporter gene and that is ligated downstream of a promoter making no change of transcriptional activity from a ligand contact with the ligand responsive transcriptional regulatory factor.

3. A cell that expresses an aryl hydrocarbon receptor gene and that comprises the following genes (a) and (b) introduced to the chromosomes thereof:
(a) a reporter gene ligated downstream of a recognition sequence for the aryl hydrocarbon receptor and a nucleotide sequence necessary for the initiation of transcription; and
(b) a reporter gene that encodes a protein distinguishable from a protein encoded by the above reporter gene and that is ligated downstream of a promoter making no change of transcriptional activity from a dioxin contact.

4. A cell that expresses an aryl hydrocarbon receptor gene and an Arnt gene and that comprises the following genes (a) and (b) introduced to the chromosomes thereof:
(a) a reporter gene ligated downstream of a recognition sequence for the aryl hydrocarbon receptor and a nucleotide sequence necessary for the initiation of transcription; and
(b) a reporter gene that encodes a protein distinguishable from a protein encoded by the above reporter gene and that is ligated downstream of a promoter making no change of transcriptional activity from a ligand contact with the ligand responsive transcriptional regulatory factor.

5. Use of a cell according to any one of claim 1, 2, 3, or 4 for evaluating the agonist or antagonist activity of a test substance against the ability of a ligand responsive transcriptional regulatory factor to promote transcription in a reporter assay measuring the amount of expression of a reporter gene that undergoes transcriptional regulation by the ligand responsive transcriptional regulatory factor.

6. A method for evaluating the agonist activity of a test substance against the ability of a ligand responsive transcriptional regulatory factor to promote transcription, which comprises the steps of:
(1) culturing a cell according to any one of claim 1, 2, 3, or 4 in the presence of a test substance, and then measuring the amounts of expression of the reporter genes in this cell;
(2) selecting a measured value of the amount of expression of the reporter gene ligated downstream of a recognition sequence for the ligand responsive transcriptional regulatory factor and a nucleotide sequence necessary for the initiation of transcription, based on a measured value of the amount of expression of the reporter gene ligated downstream of a promoter making no change of transcriptional activity from a ligand contact; and
(3) evaluating that the test substance has agonist activity against the ability of the ligand responsive transcriptional regulatory factor to promote transcription, when the measured value of the amount of expression of the reporter gene selected in step (2) is greater than the measured value of the amount of expression of this reporter gene in the absence of the test substance.

7. A method for evaluating the antagonist activity of a test substance against the ability of a ligand responsive transcriptional regulatory factor to promote transcription, which comprises the steps of:
(1) culturing a cell according to any one of claim 1, 2, 3, or 4 in the presence of a ligand for the ligand responsive transcriptional regulatory factor and a test substance, and then measuring the amounts of expression of the reporter genes in this cell;
(2) selecting a measured value of the amount of expression of the reporter gene ligated downstream of a recognition sequence for the ligand responsive transcriptional regulatory factor and a nucleotide sequence necessary for the initiation of transcription, based on a measured value of the amount of expression of the reporter gene ligated downstream of a promoter making no change of transcriptional activity from a ligand contact; and
(3) evaluating that the test substance has antagonist activity against the ability of the ligand responsive transcriptional regulatory factor to promote transcription, when the measured value of the amount of expression of the reporter gene selected in step (2) is smaller than the measured value of the amount of expression of this reporter gene under the conditions that the ligand is present but the test substance is absent.

8. A kit for measurement comprising a cell according to any one of claim 1, 2, 3, or 4.


SEQUENCE LISTING

<110> Sumitomo Chemical Co. Ltd.

<120>

<130>

<150> JP 11/002807
<151> 1999-01-08

<160> 4

<210> 1
<211> 28
<212> DNA
<213> Artificial Sequence

<220>
<223> Designed oligonucleotide primer for PCR

<400> 1
gcgctagcat ggtagcgcct tgtcagcc        28

<210> 2
<211> 28
<212> DNA
<213> Artificial Sequence

<220>

<223> Designed oligonucleotide primer for PCR

<400> 2

gcaagcttga gtactgacct agcgagag        28

<210> 3

<211> 39

<212> DNA

<213> Artificial Sequence

<220>

<223> Designed oligonucleotides to synthesize DNA containing dioxin

responsive element

<400> 3

ctcaggcatg ttgcgtgcat ccctgaggcc agccgagct        39

<210> 4

<211> 39

<212> DNA

<213> Artificial Sequence

<220>

<223> Designed oligonucleotides to synthesize DNA containing dioxin

responsive element

<400> 4

cggctggcct cagggatgca cgcaacatgc ctgagagct        39